# EUROPEAN PATENT SPECIFICATION

(11) **EP 2 342 264 B1**
(45) Date of publication and mention of the grant of the patent: **18.03.2015**
(21) Application number: 09824083.1
(22) Date of filing: 28.10.2009
(51) Int. Cl.: C12Q 1/04, G01N 33/50, G01N 33/68

(54) **METHODS AND KITS FOR THE DETERMINING THE PRESENCE OR ABSENCE OF CYANOBACTERIA TOXINS**
VERFAHREN UND KITS ZUR BESTIMMUNG DES VORHANDENSEINS ODER NICHTVORHANDENSEINS VON CYANOBAKTERIEN-TOXINEN
PROCÉDÉS ET KITS POUR LA DÉTERMINATION DE LA PRÉSENCE OU DE L'ABSENCE DE TOXINES DE CYANOBACTÉRIES

(30) Priority: 31.10.2008 US 110021 P
(43) Date of publication of application: 13.07.2011
(73) Proprietor: Waters Technologies Corporation, Milford, MA 01757 (US)
(72) Inventor: OEHRLE, Stuart, A., Cold Spring KY 41076 (US)
(74) Representative: Müller, Christian Stefan Gerd
(86) International application number: PCT/US2009/062299
(87) International publication number: WO 2010/051295

(56) References cited:
- US-A1- 2008 169 242
- XU W ET AL: "Development and application of ultra performance liquid chromatography-electrospray ionization tandem triple quadrupole mass spectrometry for determination of seven microcystins in water samples", ANALYTICA CHIMICA ACTA, ELSEVIER, AMSTERDAM, NL, vol. 626, no. 1, 19 September 2008 (2008-09-19), pages 28-36, XP024521308, ISSN: 0003-2670, DOI: 10.1016/J.ACA.2008.07.040 [retrieved on 2008-08-03]
- JUSSI MERILUOTO: "Chromatography of microcystins", ANALYTICA CHIMICA ACTA, vol. 352, no. 1-3, 1 October 1997 (1997-10-01), pages 277-298, XP55059883, ISSN: 0003-2670, DOI: 10.1016/S0003-2670(97)00131-1
- KIKUCHI ET AL: "Cylindrospermopsin determination using 2-[4-(2-hydroxyethyl)-1-piperazinyl]ethane sulfonic acid (HEPES) as the internal standard", ANALYTICA CHIMICA ACTA, ELSEVIER, AMSTERDAM, NL, vol. 583, no. 1, 30 January 2007 (2007-01-30), pages 124-127, XP022209726, ISSN: 0003-2670, DOI: 10.1016/J.ACA.2006.10.007
- FUREY AMBROSE ET AL: "The first identification of the rare cyanobacterial toxin, homoanatoxin-a, in Ireland", TOXICON, ELMSFORD, NY, US, vol. 41, no. 3, 1 March 2003 (2003-03-01), pages 297-303, XP002283647, ISSN: 0041-0101, DOI: 10.1016/S0041-0101(02)00291-X
- KUBO T ET AL: "Effective determination method for a cyanobacterial neurotoxin, beta-N-methylamino-l-alanine", TOXICON, ELMSFORD, NY, US, vol. 51, no. 7, 1 June 2008 (2008-06-01), pages 1264-1268, XP022673026, ISSN: 0041-0101, DOI: 10.1016/J.TOXICON.2008.02.015 [retrieved on 2008-02-29]
- Waters Corporation: "Acquity UPLC BEH Columns, Care and Use Manual", , 1 July 2012 (2012-07-01), pages 1-12, XP002695604, Retrieved from the Internet: URL:http://www.waters.com/webassets/cms/su pport/docs/715001371.pdf [retrieved on 2013-04-17]
- WANG. ET AL.: 'An Ultra-Performance Liquid Chromatography?Tandem Mass Spectrometry Method for Determination of Microcystins Occurrence in Surface Water in Zhejiang Province' CHINA. TOXICON vol. 49, 22 February 2007, pages 1120 - 1128, XP025318094
- GRITTI ET AL.: 'Ultra High Pressure Liquid Chromatography. Column Permeability and Changes of the Eluent Properties.' J CHROMATOGR A, [Online] vol. 1187, no. 1-2, 11 April 2008, pages 165 - 179, XP008146574 Retrieved from the Internet: <URL:http://www.ncbi.nlm.nih.gov/pubmed/183 13063?ordinalpos=1&itool=EntrezSystem2.PEnt rez.Pu bmed.Pubmed_ResultsPanel.Pubmed_SingleItemS upl.Pubmed_Discovery_ RA&linkpos=1&log $=relatedarticles&logdbfrom=pubmed> [retrieved on 2009-12-09]

## Description

### BACKGROUND OF THE INVENTION

Embodiments of the present invention are directed to kits and methods for the detection of toxins produced by cyanobacteria. Cyanobacteria are commonly found in surface fresh water. Toxic cyanobacteria blooms are problems where such blooms may cause toxins to be released in water supplies. The major cyanobacterial toxins comprise cyclic peptides, alkaloids and lipopolysaccharides.

By way of example, without limitation, the major cyclic peptides comprising cyanobacterial toxins are nodulin, microcystin-LR, microcystin RR, and microcystin YR. The formula for nodulin is set forth below:

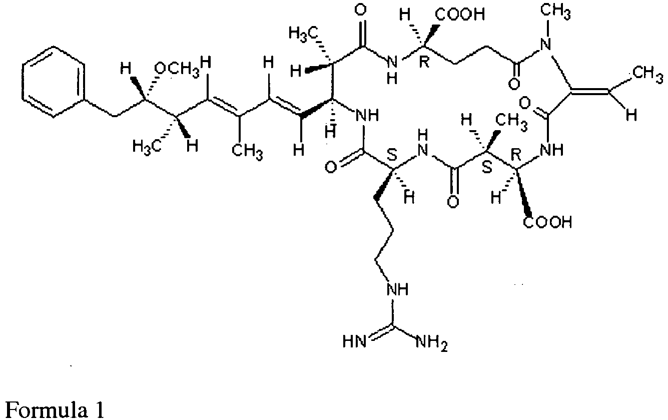

The formula for microcystin LR is set forth below:

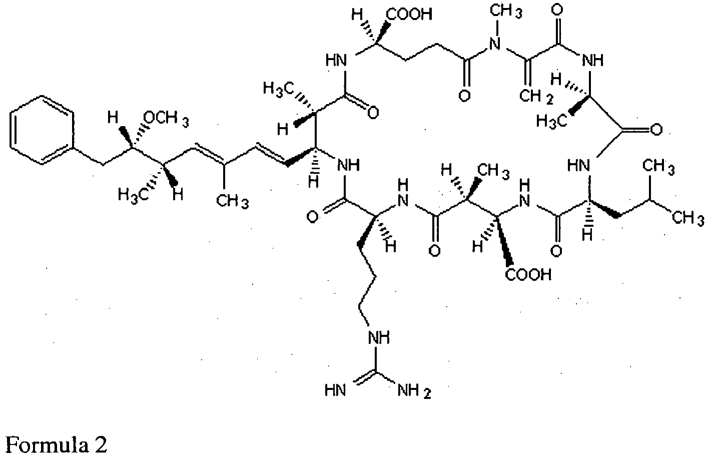

The formula for microcystin RR is set forth below:

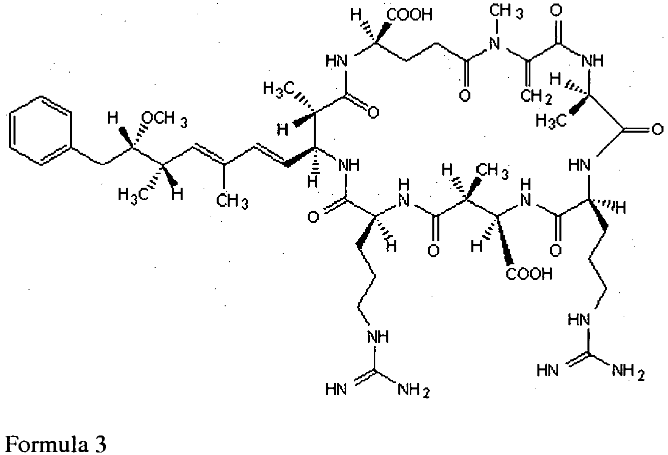

The formula for microcystin YR is set forth below:

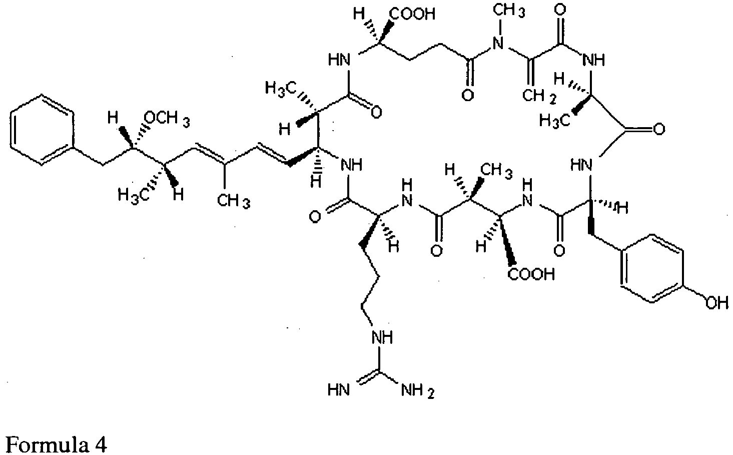

The formula for microcystin LA is set forth below:

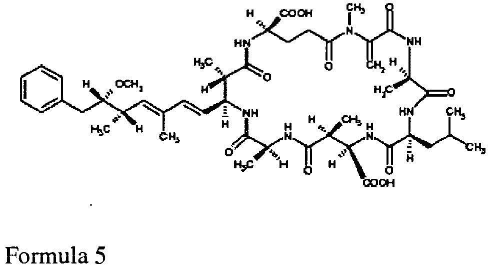

The formula for microcystin LY is set forth below:

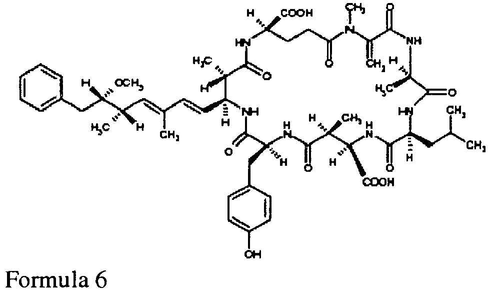

The formula for microcystin LW is set forth below:

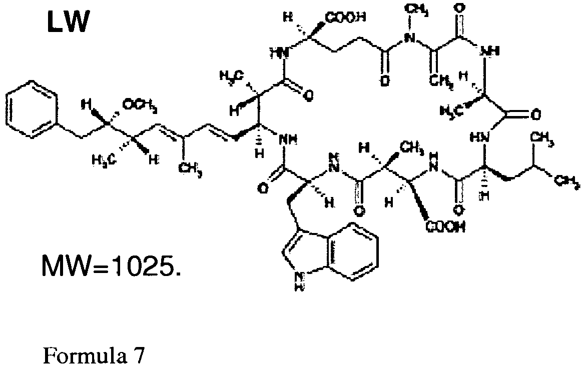

The formula for microcystin LF is set forth below:

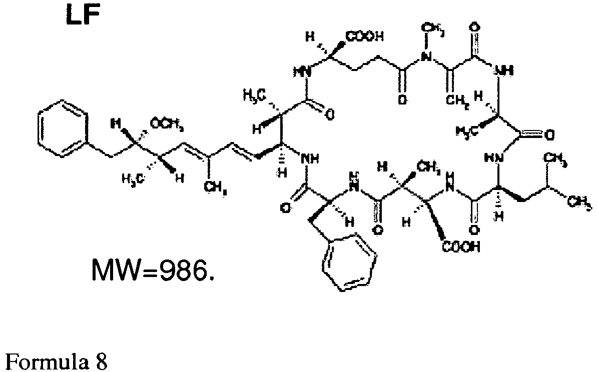

The alkaloid cyanobacterial toxins comprise, by way of example, without limitation, anaroxins and saxitoxins. Anaroxins comprise, by way of example, without limitation, anatoxin a, anatoxin a(S), homoanatoxin-a, cylindrospermopsin.

The formula for anatoxin a is set forth below:

### Figure. Chemical structures of anatoxins. Anatoxin-a, homoanatoxin-a, and anatoxin-a(s).

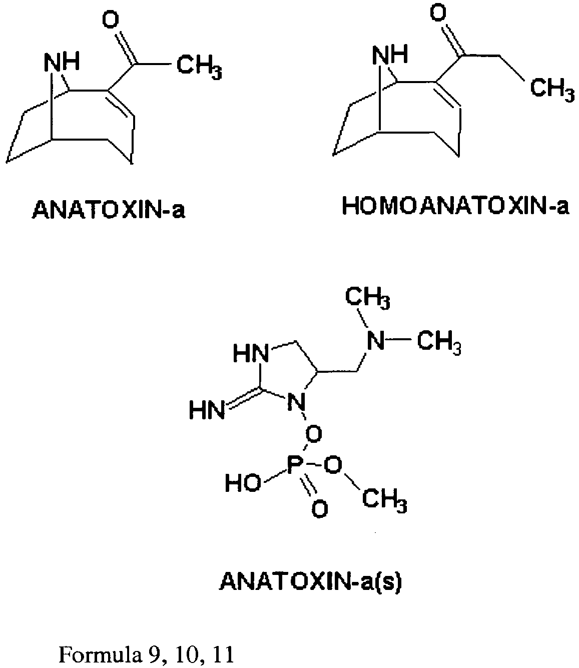

The formula for cylindrospermopsin is set forth below:

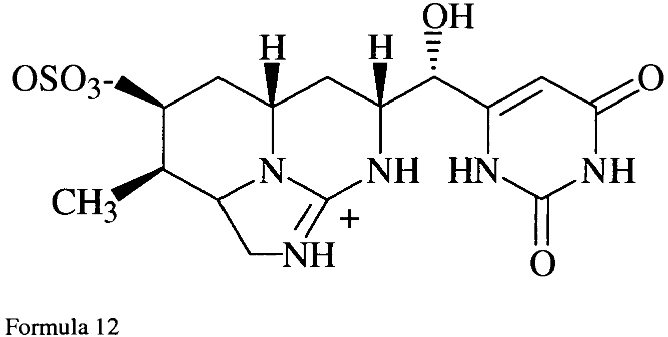

The cyanobacterial lipopolysccharides toxins are not as well characterized and appear to be less toxic than the cyclic peptides, alkaloids.

This paper will use the term "analyte" to denote a compound which one desires to determine the presence or absence of.

This paper will use the term "sample" to mean a material which one desires to test for the presence or absence of ergot alkaloids. The sample may be obtained as tissues or fluids from animals or plants. For example, without limitation, the sample may comprise leaves, seeds or other plant tissues or blood, urine, saliva or tissues obtained from animal sources.

An "extract" is a solution obtained by subjecting a sample to a solvent such that one or more compounds held in the sample are dissolved in the solution.

An "aliquot" is used to denote a subpart or fraction of a sample.

Chromatography is a method of separating compounds in a solution. Chromatography can be performed in different devices. This paper will use the term "cartridge" to refer to low pressure devices comprising a column and/or funnel in which a solid phase is placed. The sample is applied to the solid phase and passes through under low pressure or gravity. These devices are typically used to prepare a sample by removing particulates and concentrating desired compounds.

For the purpose of this paper, the term "column" will be used in the sense of a high pressure device in which solutions are forced through a solid phase matrix under pressure. The solid phase can be particulate or a porous monolith.

Mass spectrometry is used to determine the mass to charge ratio of ions formed by compounds. Mass spectrometers are used to form fragments of larger molecules and such fragments and complete ions are used to identify such compounds.

Standards are solutions with known amounts of compounds which solutions are used to compare data to data derived from non-standard samples. Standards can use compounds with labels comprising heavy isotopes which allow the operator of the mass spectrometer to differentiate between the standard and the analyte.

Interest in reliable and fast analysis of cyanobacterial toxins is needed to monitor water supplies, protect public health and fisheries. Prior to the present invention, the methods used to detect cyanobacterial toxins were not specific or sensitive to analyze for these compounds in the environment. Prior to the present invention, the methods were time consuming and labor intensive. Wang et al. (Toxicon 49 (2007), 1120-1128) describe an ultra-performance liquid chromatography-tandem mass spectrometry (UPLC-MS/MS) method for the determination of microcystins occurrence in surface water, wherein water samples are concentrated and cleaned with solid phase extraction with Oasis HLB cartridge.

### BRIEF SUMMARY OF THE INVENTION

Embodiments of the present invention are directed to reliable and fast analysis of cyanobacterial toxins. Embodiments of the present invention have utility for monitoring water supplies, protecting public health and fisheries. Embodiments of the present invention are directed to methods and kits for the detection of cyanobacterial toxins.

In a first aspect, the present invention provides a method of determining the presence or absence of cyclic peptide and alkaloid cyanobacterial toxins in a sample comprising the steps of:
preparing a water sample potentially comprising cyclic peptide and alkaloid cyanobacterial toxins to form a retained or concentrated toxin sample on a solid phase extraction device comprising a weak cationic exchange resin and a weak anionic exchange resin;
eluting or reconstituting said retained or concentrated toxin sample in a mobile phase to form a sample extract;
placing said sample extract on the head of a chromatographic column packed with particles having a mean particle size of 1 to 3 µm (microns) under pressure of 41.37 to 103.4 MPa (6,000 to 15,000 psi) to form a retained sample potentially comprising a cyclic peptide or alkaloid cyanobacterial toxin in the event said sample extract contained such cyanobacterial toxin;
eluting compounds from said retained sample potentially comprising a cyclic peptide or alkaloid cyanobacterial toxin from said chromatographic column under a gradient of organic solvent to form at least one eluted compound comprising a cyclic peptide or alkaloid cyanobacterial toxin, in the event said sample extract contained such cyanobacterial toxin; and
placing said eluted compound potentially comprising a cyclic peptide or alkaloid cyanobacterial toxin in a mass spectrometer to form a mass spectra and determining the presence or absence of said cyanobacterial toxin from the mass spectra.

In a second aspect, the present invention provides a kit for performing an analysis of a sample for the presence or absence of cyclic peptide and alkaloid cyanobacterial toxins, comprising:
standards for calibrating and facilitating the identification of one or more cyclic peptide and alkaloid cyanobacterial toxins by mass spectroscopy,
sample preparation devices for forming a sample extract, wherein said devices comprise a weak cationic exchange resin and a weak anionic exchange resin, and
a column for separating the compounds of the sample extract and upon application of a gradient releasing said cyanobacterial toxins, if present, such that said cyanobacterial toxins are released to a mass spectrometer for identification.

Preferred embodiments of the aspects of the invention are set forth in claims 2 to 8 and 10 to 12.

Embodiments of the present method can be performed from said steps of placing the sample extract on the head of a chromatographic column to eluting and placing said cyanobacterial toxin in a mass spectrometer in a time period of less than fifteen minutes, and, preferably, ten minutes.

Preferably, the mass spectrometer forms one or more fragments of the cyanobacterial toxin and the spectra of the fragments are used to identify and determine the presence or absence of the cyanobacterial toxin. Preferably, the method comprises comparing the spectra from the parent ions and fragments to those obtained with standards.

Preferably, the particle is selected from the group consisting of a bridged ethyl hybrid and high strength silica. Preferred particles have a mean average diameter of less than three micrometers (microns).

A preferred resin capable of being functionalized with weak anionic and weak cationic functional groups comprises poly(divinylbenzene-co-N-vinylpyrrolidone).

As used herein, the term "kit" refers to an article of manufacture, an assembly of parts, reagents, and components for performing a method. Kits are typically packaged in suitable packaging such a wrap, box, clam shell or bag with instructions for use.

A preferred column of the kit has a packing of particles having an average size of 1 - 3 µm (microns). A preferred particle has a chromatographic surface selected from the group consisting of a bridged ethyl hybrid and high strength silica. A preferred column has an operating pressure of 41.37 to 103.4 MPa (6,000 to 15,000 psi).

Other features and advantages of the present invention will be apparent to individuals skilled in the arts upon viewing the figures and the detailed description that follow.

### BRIEF DESCRIPTION OF THE FIGURES

Figure 1 depicts in schematic form an instrument for performing the method of the present invention;
Figure 2 depicts a kit embodying features of the present invention; and
Figure 3 depicts a separation and mass chromatogram of cyanobacterial toxins.

### DETAILED DESCRIPTION OF THE INVENTION

Embodiments of the present invention will be described in detail as methods and kits for the detection of cyclic peptide and alkaloid cyanobacterial toxins. Embodiments of the present invention have utility for monitoring water supplies, protecting public health and fisheries, as well as food testing. The descriptions that follow are preferred embodiments reflecting what the inventors now consider is the best mode to practice their invention. Such descriptions are capable of modification and alteration by those skilled in the art without departing from the teaching hereof.

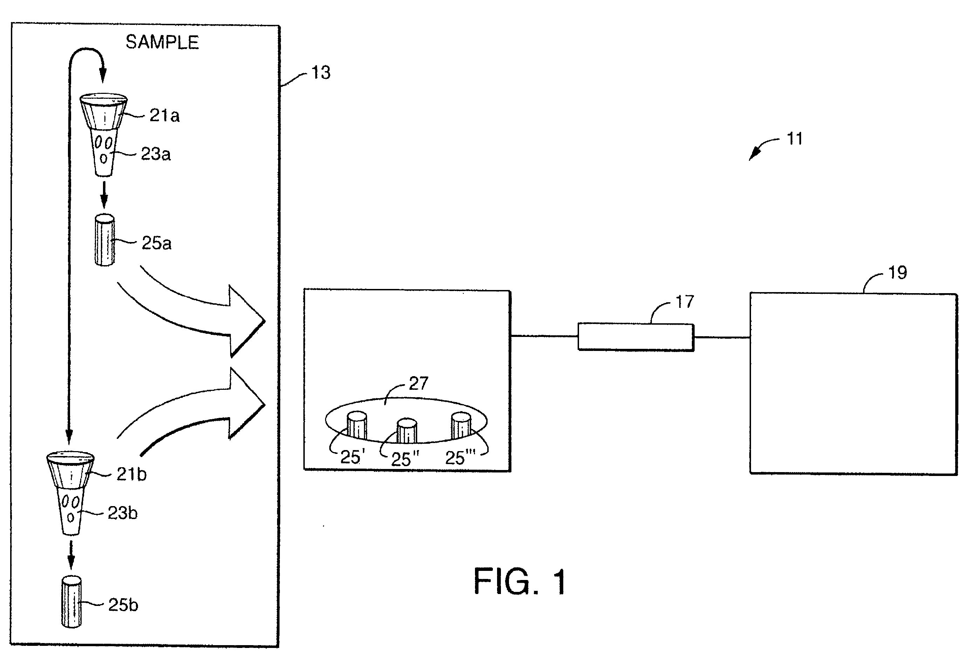

One embodiment of the present invention, directed to a method for detecting the presence or absence of cyclic peptide and alkaloid cyanobacterial toxins in a sample is depicted in schematic form in Figure 1 with respect to an instrument generally designated by the numeral 11. The instrument, generally designated by the numeral 11, has the following major elements: sample preparation means 13, a chromatography system 15, a column 17 and a mass spectrometer 19.

The method comprises the step of preparing a water sample potentially comprising cyclic peptide and alkaloid cyanobacterial toxins with respect to sample preparation means 13. As depicted, sample preparation means 13 comprises at least one sample extraction cartridge 21a, preferably two sample extraction cartridges 21a and 21b, and comprises a weak cationic exchange resin and a weak anionic exchange resin.

Sample extraction cartridges 21a and 21b are depicted in cross section. Each sample extraction cartridge 21a and 21b has a solid phase 23a and 23b. A solid phase may comprise a bed of particles or a porous monolith resin. A preferred solid phase is particular and has a surface chemistry of poly(divinylbenzene-co-N-vinylpyrrolidone). That is, the particles may totally comprise the polymer or such polymer is carried as a surface layer on a substrate that is selected from a different material. Common materials which may be used as a substrate include, by way of example, without limitation, silica, aluminium and titanium oxides and other polymeric compounds. The surface chemistry of poly(divinylbenzene-co-N-vinylpyrrolidone) allows the particles of the sample extraction column to retain cyanobacterial toxins on a water wettable surface or allow the surface to be functionalized in a manner to capture cyanobacterial toxins more selectively or efficiently.

Sample extraction cartridges having a surface chemistry of poly(divinylbenzene-co-N-vinylpyrrolidone) are sold by Waters Corporation (Milford, Massachusetts, USA) under the trademark OASIS®. Sample extraction cartridges without a surface chemistry of poly(divinylbenzene-co-N-vinylpyrrolidone) may also be used. Such sample extraction cartridges are sold by Waters Corporation (Milford, Massachusetts, USA) under the trademark SEP-PAK.

Particles having a functionalized surface are functionalized as weak cationic exchange resins and weak anionic exchange resins for the preferential or efficient capture of cyanobacterial toxins. For example, without limitation, the sample extract is formed by extracting on weak anionic exchange resin to favor alkaloid cyanobacterial toxins and formed on weak cationic exchange resins to favor cyclic peptide cyanobacterial toxins.

Thus, as depicted, the sample is divided into two or more parts with one part directed to a first sample extraction cartridge 21a having a particle bed 23a comprising a weak anionic exchange resin to favor alkaloid cyanobacterial toxins. A second sample extraction cartridge 21b has a particle bed 23b comprising a weak cationic exchange resin to favor cyclic peptide cyanobacterial toxins. Such sample extraction cartridges 21a and 21b having weak cationic or weak anionic exchange resins are sold by several venders including those sold by Waters Corporation (Milford, MA) under the trademark OASIS® WCX and OASIS® WAX.

The sample preparation means is depicted as sample extraction cartridges 21a and 21b as single well type devices with the understanding that such devices may have many forms comprising, by way of example, without limitation, single columns, cartridges and well devices as well as multiple well devices, such as 96 well plates and the like.

The method comprises the step of forming at least one sample extract with both a weak cationic exchange resin and a weak anionic exchange resin. The sample extract from sample extraction cartridge 21a is eluted and placed in a vial 25a and the sample extract from sample extraction cartridge 21b is eluted placed in vial 25b. The sample extraction cartridges 21a and 21b retain the cyanobacterial toxin and release the cyanobacterial toxin upon elution with a mobile phase in a more concentrated form. This sample extract is placed and held in vials 25a and 25b.

The vials 25a and 25b are placed in a chromatography system 15 autosampler, depicted in schematic form as a circular tray 27 holding vials 25', 25", and 25"'. Chromatography systems are well known in the art. Such chromatography systems 15 are sold by Waters Corporation (Milford, Massachusetts, USA) under the trademark ACQUITY®.

Next, as depicted in Figure 1, the method comprises the step of placing the sample extract on the head of chromatographic column 17. Chromatographic column 17 is packed with particles having a mean particle size of 1 to 3 µm (microns). Chromatographic column 17 has an operating pressure of 41.37 to 103.4 MPa (6,000 to 15,000 psi). A preferred column has particles with a chromatographic surface of a bridged ethyl hybrid composition or a high strength silica. Columns 17, having a 1.7 µm (micron) particle size, are sold by Waters Corporation (Milford, Massachusetts, USA) under the trademark ACQUITY® with a BEH designation with respect to a bridged ethyl hybrid chemistry and a HSS designation with respect to high strength silica chemistry. In the event the sample extract held in vials 25', 25" or 25"' has one or more cyanobacterial toxin, a retained cyanobacterial toxin is held on the particles until eluted under gradient conditions. The compounds of the sample are retained on the column to form one or more retained compounds.

Next, the one or more retained compounds are eluted under a gradient of organic solvent to form an eluted compound. And, in the event said sample extract contained such cyclic peptide and alkaloid cyanobacterial toxins, such eluted compound is a toxin. A preferred gradient comprises a first solvent comprising 0.1% formic acid (H₂O) and a second solvent comprising 0.1 % formic acid (acetonitrile). The gradient is applied at a flow rate of 0.1 to 1.0ml/min, and more preferably, at about 0.45ml/min over a period of approximately six minutes moving from 2% of the first solvent to 80% of the second solvent.

This eluted cyclic peptide and alkaloid cyanobacterial toxin, if present, is placed in a mass spectrometer 19 to form a mass spectra. The presence or absence of the cyanobacterial toxin is determined from the mass spectra.

Preferably, the mass spectrometer 19 forms one or more fragments of the cyanobacterial toxin. The formation of fragments in mass spectroscopy is sometimes denoted as MS/MS and is known to those skilled in the art. The spectra of the fragments are used to identify and determine the presence or absence of a cyanobacterial toxin. Mass spectrometers are sold by several venders including Waters Corporation (Milford, Massachusetts, USA) under the trademark MICROMASS® TQD.

The identification of the cyclic peptide and alkaloid cyanobacterial toxin, if present, is facilitated by placing one or more standards comprising a known labeled cyanobacterial toxin or closely related compound on the head of a column to be retained and eluted in the manner of sample cyanobacterial toxin. The eluted standard cyanobacterial toxin is placed in a mass spectrometer 19 to form a known spectra of the standard cyanobacterial toxin to which sample spectra are compared. Such labeled cyanobacterial toxin or closely related compound is used in a deuterated form known to individuals skilled in the art. The examples feature cyclo(Arg-Ala-Asp-D-Phe-Val) and [Leu5]-Enkephalin.

The small particle column and high pressure performance of the chromatography system allow the method steps of placing the sample extract on the head of a chromatographic column, eluting and placing the cyanobacterial toxin in a mass spectrometer to be performed in a time period of three to fifteen minutes, and routinely in a period of approximately eight to nine minutes.

Preferably, the mass spectrometer forms one or more fragments of the cyanobacterial toxin and the spectra of the fragments are used to identify and determine the presence or absence of the cyanobacterial toxin. Preferably, the method comprises comparing the spectra from the parent ions and fragments to those obtained with standards.

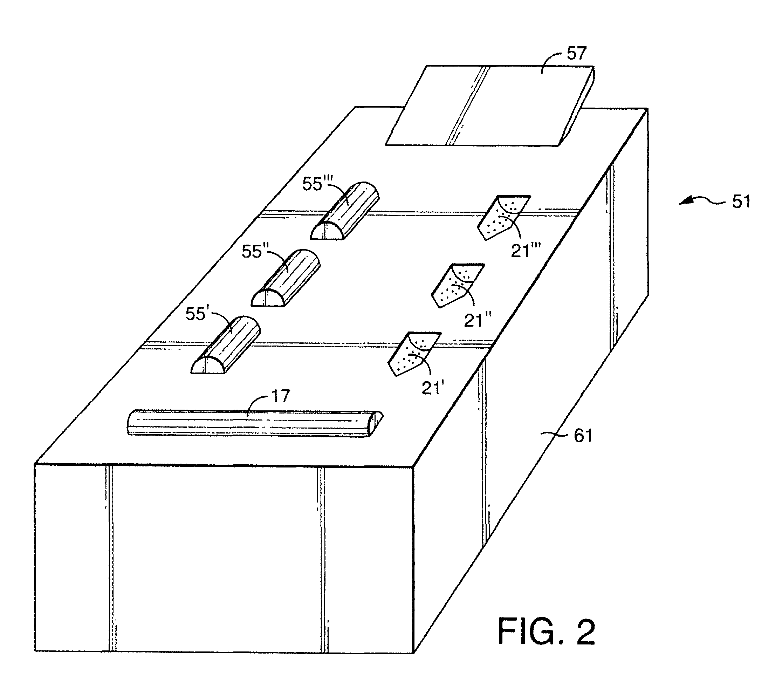

Turning now to Figure 2, a kit embodying features of the present invention, generally designated by the numeral 51, is illustrated. The kit is a collection of parts and reagents bundled together with suitable packaging and instructions for their use in the method described above. Kit 51 comprises one or more standard vials, of which three are depicted and designated 55', 55" and 55"', containing standard solutions for calibrating and facilitating the identification of one or more cyclic peptide and alkaloid cyanobacterial toxins by mass spectroscopy. The kit 51 further comprises one or more sample preparation devices in the form of extraction cartridges, of which three are depicted and designated 21', 21", and 21"' for forming a sample extract. The kit further comprises a column 17 for separating the compounds of the sample extract and upon application of a gradient releasing the cyanobacterial toxins, if present, such that the cyanobacterial toxins are released to a mass spectrometer for identification. The kit 51 further comprises instructions 57 for the use of these parts and reagents in the method as previously described. The kit is depicted with suitable packaging, which is known in the art, and may comprise plastic wraps and bubble shells, boxes, wrapping and the like.

Further features of the present invention are described with respect to the following examples.

### Example 1

### Multi column solid phase extraction (SPE) using mixed mode cartridges

This discussion is focused on an analysis of cylindrospermopsin, anatoxin-a and microcystins from lake or process water or water extracts from filters or other surfaces.

### Method Summary:

Cartridges are initially conditioned, equilibrated and loaded in series (OASIS®WCX on top followed by OASIS®WAX cartridge with a union connecting the two). Once loaded they are separated and processed individually and run as two separate runs.

### Protocol:

**WAX** (6cc 150mg Waters part number 186002493) - This is for cylindrospermopsin enrichment (this is on the bottom).

| | |
|---|---|
| Condition: | 3mL MeOH |
| Equilibrate: | 5mL H₂O |
| Load: | Up to 500mL of sample water (pH the water to pH 5.5 with formic acid) |

### SEPARATE COLUMNS AND CONTINUE ON EACH SEPERATELY.

| | |
|---|---|
| Wash: | 2mL 1% formic acid in DI H₂O |
| Wash 2: | 2mL MeOH |
| Elute: | 3mL 1 % ammonium hydroxide in DI H₂O |

Run "as is" or evaporate to dryness and reconstitute in mobile phase.

WCX (6cc 150mg Waters part number 186002498) - This is for anatoxin-a, and microcystins enrichment (this is on the top).

| | |
|---|---|
| Condition: | 3mL MeOH |
| Equilibrate: | 5mL H₂O |
| Load: | Up to 500mL of sample water (pH the water to pH 5.5 with formic acid) |

### SEPARATE COLUMNS AND CONTINUE ON EACH SEPERATELY.

| | |
|---|---|
| Wash: | 2mL pH 9 ammonium hydroxide or ammonium bicarbonate in DI H₂O |
| Wash 2: | 2mL MeOH |
| Elute: | 3mL 1% formic acid in DI H₂O |

These cartridges are run "as is" or evaporated to dryness and reconstituted in mobile phase.

### Example 2

### Anatoxin-a, cylindrospermopsin, and Microcystins by extreme pressure high performance liquid chromatography/MS/MS

This example features the separation and mass spectral analysis of anatoxin-a, cylindrospermopsin, and several microcystins by high performance liquid chromatography and mass spectrometry.
Column Used: HSS T3 2.1X100mm @35C
   or BEH C18 2.1X100mm @35C
Solvent A: 0.1% formic acid in H₂O
Solvent B: 0.1% formic acid in acetonitrile

### [Gradient Table]

| **Time(min)** | **Flow Rate** | **%A** | **%B** | **Curve** |
|---|---|---|---|---|
| Initial | 0.450 | 98.0 | 2.0 | ---- |
| 0.80 | 0.450 | 98.0 | 2.0 | 6 |
| 9.00 | 0.450 | 30.0 | 70.0 | 6 |
| 9.05 | 0.450 | 20.0 | 80.0 | 6 |
| 9.90 | 0.450 | 20.0 | 80.0 | 6 |
| 9.91 | 0.450 | 98.0 | 2.0 | 6 |
| 12.00 | 0.450 | 98.0 | 2.0 | 6 |

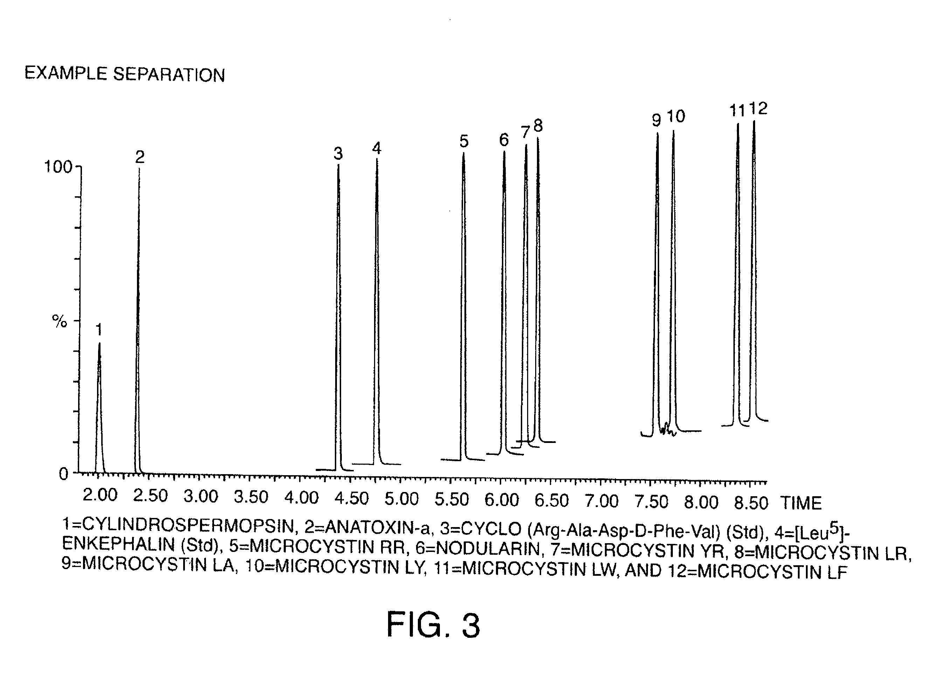

These results are set forth in Figure 3. These results suggest that the compounds identified can be separated and identified in less than nine minutes.

### TQD Conditions (MS/MS)

### Function 1 - Cylindrospermopsin

| | |
|---|---|
| Retention window (mins): | 1.000 to 2.220 |
| Ionization mode: | ES+ |
| Data type: | MRM data |
| Function type: | MRM of 3 channels |

| Chan Reaction | Dwell(secs) | Cone Volt. | Col.Energy | Delay(secs) | Compound |
|---|---|---|---|---|---|
| 1 : 416.20 > 176.23 | 0.060 | 45.0 | 39.0 | Auto | Cylindro (C1) |
| 2 : 416.20 > 194.25 | 0.060 | 45.0 | 37.0 | Auto | Cylindro (Q) |
| 3 : 416.20 > 416.20 | 0.060 | 45.0 | 5.0 | Auto | Cylindro (C2) |

### Function 2 - Anatoxin-a

Retention window (mins): 2.220 to 3.000
Ionization mode: ES+
Data type: MRM data
Function type: MRM of 3 channels

| Chan Reaction | Dwell(secs) | ConeVolt. | Col.Energy | Delay(secs) | Compound |
|---|---|---|---|---|---|
| 1 : 166.13 > 43.03 | 0.060 | 20.0 | 23.0 | Auto | Anatoxin (Q) |
| 2 : 166.13 > 149.14 | 0.060 | 20.0 | 15.0 | Auto | Anatoxin (C1) |
| 3 : 166.13 > 166.13 | 0.060 | 25.0 | 5.0 | Auto | Anatoxin (C2) |

### Function 3 - Cyclo(Arg-Ala-Asp-D-Phe-Val) (used as an Internal Standard)

Retention window (mins): 4.150 to 4.550
Ionization mode: ES+
Data type: MRM data
Function type: MRM of 3 channels

| Chan Reaction | Dwell(secs) | Cone Volt. | Col.Energy | Delay(secs) | Compound |
|---|---|---|---|---|---|
| 1 : 589.47 > 72.77 | 0.050 | 55.0 | 73.0 | Auto | Cyclo (IS)-C2 |
| 2 : 589.47 > 120.16 | 0.050 | 55.0 | 57.0 | Auto | Cyclo (IS) |
| 3 : 589.47 > 589.47 | 0.050 | 55.0 | 5.0 | Auto | Cyclo (IS)-C1 |

### Function 4 - [Leu5]-Enkephalin (used as an Internal Standard)

Retention window (mins): 4.500 to 5.000
Ionization mode: ES+
Data type: MRM data
Function type: MRM of 3 channels

| Chan Reaction | Dwell(secs) | Cone Volt. | Col.Energy | Delay(secs) | Compound |
|---|---|---|---|---|---|
| 1 : 556.42 > 120.16 | 0.050 | 35.0 | 53.0 | Auto | Enk (IS)-C2 |
| 2 : 556.42 > 136.17 | 0.050 | 35.0 | 53.0 | Auto | Enk (IS) |
| 3 : 556.42 > 556.42 | 0.050 | 35.0 | 5.0 | Auto | Enk (IS)-C1 |

### Function 5 - Microcystin RR

Retention window (mins): 5.400 to 6.000
Ionization mode: ES+
Data type: MRM data
Function type: MRM of 3 channels

| Chan Reaction | Dwell(secs) | Cone Volt. | Col.Energy | Delay(secs) | Compound |
|---|---|---|---|---|---|
| 1 : 519.99 > 135.10 | 0.070 | 45.0 | 32.0 | Auto | RR (Q) |
| 2 : 519.99 > 519.99 | 0.070 | 45.0 | 5.0 | Auto | RR (C1) |
| 3 : 1038.69 > 135.10 | 0.070 | 90.0 | 80.0 | Auto | RR (C2) |

### Function 6 - Microcystin YR

Retention window (mins): 6.100 to 6.400
Ionization mode: ES+
Data type: MRM data
Function type: MRM of 2 channels

| Chan Reaction | Dwell(secs) | Cone Volt. | Col.Energy | Delay(secs) | Compound |
|---|---|---|---|---|---|
| 1 : 1045.60 > 135.18 | 0.050 | 95.0 | 50.0 | Auto | YR (Q) |
| 2 : 1045.60 > 1045.60 | 0.050 | 95.0 | 5.0 | Auto | YR (C1) |

### Function 7 - Microcystin LR

Retention window (mins): 6.150 to 6.550
Ionization mode: ES+
Data type: MRM data
Function type: MRM of 2 channels

| Chan Reaction | Dwell(secs) | Cone Volt. | Col.Energy | Delay(secs) | Compound |
|---|---|---|---|---|---|
| 1 : 995.66 > 135.11 | 0.050 | 85.0 | 50.0 | Auto | LR (Q) |
| 2 : 995.66 > 995.66 | 0.050 | 50.0 | 5.0 | Auto | LR (C1) |

### Function 8 - Microcystin LA

Retention window (mins): 7.400 to 7.750
Ionization mode: ES+
Data type: MRM data
Function type: MRM of 2 channels

| Chan Reaction | Dwell(secs) | Cone Volt. | Col.Energy | Delay(secs) | Compound |
|---|---|---|---|---|---|
| 1 : 910.57 > 135.11 | 0.050 | 45.0 | 50.0 | Auto | LA (Q) |
| 2 : 910.57 > 910.57 | 0.050 | 45.0 | 5.0 | Auto | LA (C1) |

### Function 9 - Microcystin LY

Retention window (mins): 7.600 to 8.000
Ionization mode: ES+
Data type: MRM data
Function type: MRM of 2 channels

| Chan Reaction | Dwell(secs) | Cone Volt. | Col.Energy | Delay(secs) | Compound |
|---|---|---|---|---|---|
| 1 : 1002.63 > 135.18 | 0.050 | 50.0 | 50.0 | Auto | LY (Q) |
| 2 : 1002.63 > 1002.63 | 0.050 | 50.0 | 5.0 | Auto | LY (C) |

### Function 10 - Microcystin LW

Retention window (mins): 8.200 to 8.700
Ionization mode: ES+
Data type: MRM data
Function type: MRM of 2 channels

| Chan Reaction | Dwell(secs) | Cone Volt. | Col.Energy | Delay(secs) | Compound |
|---|---|---|---|---|---|
| 1 : 1025.63 > 135.05 | 0.070 | 45.0 | 42.0 | Auto | LW (Q) |
| 2 : 1025.63 > 1025.63 | 0.070 | 45.0 | 5.0 | Auto | LW (C1) |

### Function 11 - Microcystin LF

Retention window (mins): 8.430 to 8.700
Ionization mode: ES+
Data type: MRM data
Function type: MRM of 2 channels

| Chan Reaction | Dwell(secs) | Cone Volt. | Col.Energy | Delay(secs) | Compound |
|---|---|---|---|---|---|
| 1 : 986.63 > 135.05 | 0.050 | 40.0 | 50.0 | Auto | LF (Q) |
| 2 : 986.63 > | | | | | |

## Claims

1. A method of determining the presence or absence of cyclic peptide and alkaloid cyanobacterial toxins in a sample comprising the steps of:
preparing a water sample potentially comprising cyclic peptide and alkaloid cyanobacterial toxins to form a retained or concentrated toxin sample on a solid phase extraction device comprising a weak cationic exchange resin and a weak anionic exchange resin;
eluting or reconstituting said retained or concentrated toxin sample in a mobile phase to form a sample extract;
placing said sample extract on the head of a chromatographic column packed with particles having a mean particle size of 1 to 3 µm (microns) under pressure of 41.37 to 103.4 MPa (6,000 to 15,000 psi) to form a retained sample potentially comprising a cyclic peptide or alkaloid cyanobacterial toxin in the event said sample extract contained such cyanobacterial toxin;
eluting compounds from said retained sample potentially comprising a cyclic peptide or alkaloid cyanobacterial toxin from said chromatographic column under a gradient of organic solvent to form at least one eluted compound comprising a cyclic peptide or alkaloid cyanobacterial toxin, in the event said sample extract contained such cyanobacterial toxin; and
placing said eluted compound potentially comprising a cyclic peptide or alkaloid cyanobacterial toxin in a mass spectrometer to form a mass spectra and determining the presence or absence of said cyanobacterial toxin from the mass spectra.

2. The method of claim 1 wherein said mass spectrometer forms one or more fragments of the cyanobacterial toxin and said spectra of said fragments is used to identify and determine the presence or absence of said cyanobacterial toxin.

3. The method of claim 1 or 2 wherein said steps of placing said sample extract on the head of a chromatographic column, eluting and placing said cyanobacterial toxin in a mass spectrometer is performed in a time period of less than fifteen minutes.

4. The method of claim 1 wherein said particle is selected from the group consisting of a bridged ethyl hybrid and high strength silica.

5. The method of claim 1 wherein said particles have a mean average diameter of less than two micrometers (microns).

6. The method of claim 1 wherein said sample extract is formed by extracting alkaloid cyanobacterial toxins on the weak anionic exchange resin.

7. The method of claim 1 wherein said sample extract is formed by extracting cyclic peptide cyanobacterial toxins on the weak cationic exchange resin.

8. The method of claim 1 wherein said solid phase extraction device has particles comprising poly(divinylbenzene-co-N-vinylpyrrolidone).

9. A kit for performing an analysis of a sample for the presence or absence of cyclic peptide and alkaloid cyanobacterial toxins, comprising:
standards for calibrating and facilitating the identification of one or more cyclic peptide and alkaloid cyanobacterial toxins by mass spectroscopy,
sample preparation devices for forming a sample extract, wherein said devices comprise a weak cationic exchange resin and a weak anionic exchange resin, and
a column for separating the compounds of the sample extract and upon application of a gradient releasing said cyanobacterial toxins, if present, such that said cyanobacterial toxins are released to a mass spectrometer for identification.

10. The kit of claim 9 wherein said column is packed with particles having an average particle size of 1 - 3 µm (microns).

11. The kit of claim 10 wherein said particle has a chromatographic surface selected from the group consisting of a bridged ethyl hybrid and high strength silica.

12. The kit of claim 11 wherein said column has an operating pressure of 41.37 to 103.4 MPa (6,000 to 15,000 psi).

## Patentansprüche

1. Verfahren zum Bestimmen des Vorhandenseins oder der Abwesenheit von cyclischen Peptid- und Alkaloid-Cyanobakterien-Toxinen in einer Probe, umfassend die Schritte:
Herstellen einer Wasserprobe, die möglicherweise cyclische Peptid- und Alkaloid-Cyanobakterien-Toxine umfasst, um eine zurückgehaltene oder konzentrierte Toxinprobe auf einer Festphasenextraktionsvorrichtung, die ein Harz eines schwachen Kationenaustauschers und ein Harz eines schwachen Anionenaustauschers umfasst, auszubilden;
Eluieren oder Wiederherstellen der zurückgehaltenen oder konzentrierten Toxinprobe in einer mobilen Phase, um einen Probenextrakt auszubilden;
Platzieren des Probenextraktes auf den Kopf einer chromatographischen Säule, die mit Partikeln gepackt ist, die eine mittlere Partikelgröße von 1 bis 3 µm (Mikrons) aufweisen, unter einem Druck von 41,37 bis 103,4 MPa (6 000 bis 15 000 psi), um eine zurückgehaltene Probe auszubilden, die möglicherweise ein cyclisches Peptid- oder Alkaloid-Cyanobakterien-Toxin umfasst, falls der Probenextrakt ein solches Cyanobakterien-Toxin enthielt;
Eluieren von Verbindungen aus der zurückgehaltenen Probe, die möglicherweise ein cyclisches Peptid- oder Alkaloid-Cyanobakterien-Toxin umfasst, von der chromatographischen Säule unter einem Gradienten eines organischen Lösungsmittels, um mindestens eine eluierte Verbindung auszubilden, die ein cyclisches Peptid- oder Alkaloid-Cyanobakterien-Toxin umfasst, falls der Probenextrakt ein solches Cyanobakterien-Toxin enthielt; und
Platzieren der eluierten Verbindung, die möglicherweise ein cyclisches Peptid- oder Alkaloid-Cyanobakterien-Toxin umfasst, in ein Massenspektrometer, um ein Massenspektrum auszubilden, und Bestimmen des Vorhandenseins oder der Abwesenheit des Cyanobakterien-Toxins aus dem Massenspektrum.

2. Verfahren nach Anspruch 1, wobei das Massenspektrometer ein oder mehrere Fragmente des Cyanobakterien-Toxins bildet und das Spektrum der Fragmente zum Identifizieren und Bestimmen des Vorhandenseins oder der Abwesenheit des Cyanobakterien-Toxins verwendet wird.

3. Verfahren nach Anspruch 1 oder 2, wobei die Schritte eines Platzierens des Probenextraktes auf den Kopf einer chromatographischen Säule, eines Eluierens und eines Platzierens des Cyanobakterien-Toxins in ein Massenspektrometer in einer Zeitspanne von weniger als fünfzehn Minuten durchgeführt wird.

4. Verfahren nach Anspruch 1, wobei das Partikel ausgewählt wird aus der Gruppe, bestehend aus verbrückter Ethylgruppe-Hybrid-Silica ("bridged ethyl hybrid silica") und Silica hoher Festigkeit ("high strength silica").

5. Verfahren nach Anspruch 1, wobei die Partikel einen gewogenen Mittelwert des Durchmessers von weniger als 2 Mikrometer (Mikrons) aufweisen.

6. Verfahren nach Anspruch 1, wobei der Probenextrakt durch Extrahieren von Alkaloid-Cyanobakterien-Toxinen auf einem Harz eines schwachen Anionenaustauschers ausgebildet wird.

7. Verfahren nach Anspruch 1, wobei der Probenextrakt durch Extrahieren von cyclischen Peptid-Cyanobakterien-Toxinen auf einem Harz eines schwachen Kationenaustauschers ausgebildet wird.

8. Verfahren nach Anspruch 1, wobei die Festphasenextraktionsvorrichtung Partikel aufweist, die Poly(divinylbenzol-co-N-vinylpyrrolidon) umfassen.

9. Kit zum Durchführen einer Analyse einer Probe hinsichtlich des Vorhandenseins oder der Abwesenheit von cyclischen Peptid- und Alkaloid-Cyanobakterien-Toxinen, umfassend:
Standards zum Kalibrieren und Ermöglichen der Identifizierung eines oder mehrerer cyclischer Peptid- und Alkaloid-Cyanobakterien-Toxine durch Massenspektroskopie,
Probenherstellungsvorrichtungen zum Ausbilden eines Probenextraktes, wobei die Vorrichtungen ein Harz eines schwachen Kationenaustauschers und ein Harz eines schwachen Anionenaustauschers umfassen, und
eine Säule zum Auftrennen der Verbindungen des Probenextraktes und, nach Anwendung eines Gradienten, Freisetzen der Cyanobakterien-Toxine, falls vorhanden, so dass die Cyanobakterien-Toxine zur Identifizierung an ein Massenspektrometer freigesetzt werden.

10. Kit nach Anspruch 9, wobei die Säule mit Partikeln gepackt ist, die eine mittlere Partikelgröße von 1 - 3 µm (Mikrons) aufweisen.

11. Kit nach Anspruch 10, wobei das Partikel eine chromatographische Oberfläche aufweist, die ausgewählt wird aus der Gruppe, bestehend aus verbrückter Ethylgruppe-Hybrid-Silica und Silica hoher Festigkeit.

12. Kit nach Anspruch 11, wobei die Säule einen Betriebsdruck von 41,37 bis 103,4 MPa (6 000 bis 15 000 psi) aufweist.

## Revendications

1. Procédé pour la détermination de la présence ou l'absence de toxines cyanobactériennes de types peptide cyclique et alcaloïde dans un échantillon, comprenant les étapes de:
préparation d'un échantillon d'eau comprenant potentiellement des toxines cyanobactériennes de types peptide cyclique et alcaloïde pour former un échantillon de toxine conservé ou concentré sur un dispositif d'extraction en phase solide comprenant une résine échangeuse cationique faible et une résine échangeuse anionique faible;
élution ou reconstitution dudit échantillon de toxine conservé ou concentré dans une phase mobile pour former un extrait d'échantillon;
placement dudit extrait d'échantillon en tête d'une colonne de chromatographie garnie avec des particules ayant une taille de particules moyenne de 1 à 3 µm (micromètres) sous une pression de 41,37 à 103,4 MPa (6.000 à 15.000 psi) pour former un échantillon conservé comprenant potentiellement une toxine cyanobactérienne de type peptide cyclique ou alcaloïde dans le cas où ledit extrait d'échantillon contenait une telle toxine cyanobactérienne;
élution de composés dudit échantillon conservé, comprenant potentiellement une toxine cyanobactérienne de type peptide cyclique ou alcaloïde à partir de ladite colonne chromatographique sous un gradient de solvant organique pour former au moins un composé élué comprenant une toxine cyanobactérienne de type peptide cyclique ou alcaloïde, dans le cas où ledit extrait d'échantillon contenait une telle toxine cyanobactérienne; et
le placement dudit composé élué comprenant potentiellement une toxine cyanobactérienne de type peptide cyclique ou alcaloïde dans un spectromètre de masse pour former un spectre de masse et la détermination de la présence ou de l'absence de ladite toxine cyanobactérienne à partir du spectre de masse.

2. Procédé selon la revendication 1, dans lequel ledit spectromètre de masse forme un ou plusieurs fragments de la toxine cyanobactérienne et ledit spectre desdits fragments est utilisé pour l'identification et la détermination de la présence ou de l'absence de ladite toxine cyanobactérienne.

3. Procédé selon la revendication 1 ou 2, dans lequel lesdites étapes de placement dudit extrait d'échantillon en tête d'une colonne chromatographique, d'élution et de placement de ladite toxine cyanobactérienne dans un spectromètre de masse sont réalisées sur une période de temps de moins de quinze minutes.

4. Procédé selon la revendication 1, dans lequel ladite particule est choisie dans le groupe consistant en un hybride éthylique ponté et de la silice à résistance élevée.

5. Procédé selon la revendication 1, dans lequel lesdites particules ont un diamètre moyen inférieur à deux micromètres (microns).

6. Procédé selon la revendication 1, dans lequel ledit extrait d'échantillon est formé par extraction de toxines cyanobactériennes de type alcaloïde sur la résine échangeuse anionique faible.

7. Procédé selon la revendication 1, dans lequel ledit extrait d'échantillon est formé par extraction de toxines cyanobactériennes de type peptide cyclique sur la résine échangeuse cationique faible.

8. Procédé selon la revendication 1, dans lequel ledit dispositif d'extraction en phase solide a des particules comprenant de la poly(divinylbenzène-co-N-vinylpyrrolidone).

9. Kit pour la réalisation d'une analyse d'un échantillon pour la présence ou l'absence de toxines cyanobactériennes de type peptide cyclique ou alcaloïde, comprenant:
des étalons pour le calibrage et pour faciliter l'identification d'une ou plusieurs toxines cyanobactériennes de type peptide cyclique et alcaloïde par spectroscopie de masse,
des dispositifs de préparation d'échantillon pour former un extrait d'échantillon, tandis que lesdits dispositifs comprennent une résine échangeuse cationique faible et une résine échangeuse anionique faible, et
une colonne pour la séparation des composés de l'extrait d'échantillon et, lors de l'application d'un gradient libérant lesdites toxines cyanobactériennes, si elles sont présentes, de telle manière que lesdites toxines cyanobactériennes sont libérées dans un spectromètre de masse pour identification.

10. Kit selon la revendication 9, dans lequel ladite colonne est garnie avec des particules ayant une taille de particules moyenne de 1 - 3 µm (micromètres).

11. Kit selon la revendication 10, dans lequel ladite particule a une surface chromatographique choisie dans le groupe consistant en un hybride éthylique ponté et de la silice à résistance élevée.

12. Kit selon la revendication 11, dans lequel ladite colonne a une pression opérationnelle de 41,37 à 103,4 MPa (6.000 à 15.000 psi).
